# EUROPÄISCHE PATENTSCHRIFT

(11) **EP 2 299 899 B1**
(45) Veröffentlichungstag und Bekanntmachung des Hinweises auf die Patenterteilung: **04.01.2012**
(21) Anmeldenummer: 09742081.4
(22) Anmeldetag: 06.05.2009
(51) Int. Cl.: A61B 5/00, B01J 3/00, G01N 21/86, G01N 33/487

(54) **DIAGNOSTISCHE BANDEINHEIT UND DIAGNOSTISCHES MESSSYSTEM**
DIAGNOSTIC TAPE UNIT AND DIAGNOSTIC MEASURING SYSTEM
ENSEMBLE BANDE DIAGNOSTIQUE ET SYSTEME DE MESURE DIAGNOSTIQUE

(30) Priorität: 06.05.2008 EP 08155742
(43) Veröffentlichungstag der Anmeldung: 30.03.2011
(73) Patentinhaber: F. Hoffmann-La Roche AG, 4070 Basel (CH); Roche Diagnostics GmbH, 68305 Mannheim (DE)
(72) Erfinder: SEELIG, Peter, 60318 Frankfurt (Main) (DE); LEININGER, Helmut, 68259 Mannheim (DE)
(74) Vertreter: Pfiz, Thomas
(86) Internationale Anmeldenummer: PCT/EP2009/055450
(87) Internationale Veröffentlichungsnummer: WO 2009/135863

(56) Entgegenhaltungen:
- WO-A-01/48461
- WO-A-2004/056269

## Beschreibung

Die Erfindung betrifft eine Diagnostische Bandeinheit, insbesondere Bandkassette für Blutzuckertests, mit einem als Bandwickel auf einer Spule aufgewickelten oder aufwickelbaren Testband, das aus einem Transportband und einer Vielzahl von darauf aufgebrachten Testelementen besteht, wobei die Testelemente eine analytische Reagenzschicht, eine die Reagenzschicht tragende Trägerfolie und ein die Trägerfolie mit dem Transportband verbindendes Klebebandstück aufweisen, und wobei die Reagenzschicht an ihrer von der Trägerfolie abgewandten Vorderseite zum Aufbringen einer Probensubstanz vorgesehen ist. Die Erfindung betrifft weiter ein Messsystem zum Einsatz einer solchen Bandeinheit.

Solche Bandeinheiten wurden vor allem für Blutzuckertests konzipiert, um gegenüber den am Markt befindlichen Teststreifensystemen die Benutzerfreundlichkeit weiter zu verbessern. So kann im Sinne einer vereinfachten Handhabung auf einem aufrollbaren Transportband eine hohe Anzahl von Testelementen kompakt bevorratet und durch den Bandtransport nach Gebrauch auch wieder entsorgt werden. Zweckmäßig ist eine solche Bandeinheit in Form einer Kassette als Verbrauchsmaterial in ein Handgerät einsetzbar, um dem Benutzer weitgehend automatisch ablaufende Selbsttests zu ermöglichen.

Bei den herkömmlichen trockenchemischen Teststreifen werden Reagenzfelder auf einen vergleichsweise dicken Reagenzträger aufgebracht. Bei einem solchen Einschicht-System gestaltet sich eine reflexionsoptische Vermessung relativ problemlos. Ein in Form eines Bandwickels aufgespultes Testband mit einer Vielzahl von Tests lässt sich damit jedoch nicht realisieren.

Ausgehend hiervon liegt der Erfindung die Aufgabe zugrunde, die im Stand der Technik bekannten Bandkonzepte, wie z.B. aus WO -A- 2004/056269 bekannt, weiter zu entwickeln und eine auch als Massenprodukt für eine zuverlässige Messung konzipierte Bandeinheit der eingangs angegebenen Art sowie ein Messsystem dafür anzugeben.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen vorderseitigen Probenauftrag und eine rückseitige Vermessung bei einem Testband mit voneinander beabstandeten Testfeldern zu ermöglichen. Dementsprechend wird erfindungsgemäß vorgeschlagen, dass die Testelemente im Verbund mit dem lichtdurchlässigen Transportband jeweils ein optisches Mehrschichtsystem für eine rückseitige reflexionsphotometrische Vermessung der Reagenzschicht bilden. Durch den mehrschichtigen Aufbau ist es möglich, relativ dünne vorgefertigte Testträger in einen Bandwickel zu integrieren, wobei durch den einheitlichen optischen Mehrschichtverbund eine messtechnische Erfassung der Remission an einem freiliegenden Testbandabschnitt unabhängig von der Handhabungsseite erfolgen kann.

Um ein Messsignal in der für diagnostische Anwendungen geforderten Güte zu erhalten, sind der Brechungsindex und/oder die Transmission und/oder die Trübung der durch die Trägerfolie, das Klebebandstück und das Transportband gebildeten Schichten des Mehrschichtsystems innerhalb vorgegebener Toleranzen aufeinander abgestimmt. Besonders günstig ist es in diesem Zusammenhang, wenn der Brechungsindex des Transportbands, des Trägerbands und des Klebebandstücks jeweils zwischen 1,4 und 1,7, vorzugsweise 1,5 bis 1,6 beträgt.

Eine weitere Verringerung von Störeinflüssen lässt sich dadurch erreichen, dass die einzelnen Schichten des Mehrschichtsystems eine Brechungsindexdifferenz von maximal 0,2 und vorzugsweise weniger als 0,1 aufweisen.

Hinsichtlich einer Optimierung der durch das Mehrschichtsystem insgesamt realisierbaren Parameter ist es vorteilhaft, wenn der Gesamtbrechungsindex etwa 1,5 beträgt. Um Reflexionseffekte in einem unkritischen Bereich zu halten, sollte die Abweichung im Brechungsindex möglichst kleiner 0,1 betragen.

Weitere Verbesserungen lassen sich dadurch erzielen, dass das Transportband, das Klebebandstück und die Trägerfolie im sichtbaren Wellenlängenbereich eine Transmission von jeweils mehr als 80%, vorzugsweise 85% bis 92% besitzen und dass die Gesamttransmission des Mehrschichtsystems im sichtbaren Wellenlängenbereich mindestens 80% beträgt.

Für eine reproduzierbare Messung ist es auch von besonderer Bedeutung, dass die Transmissionstoleranz für die Gesamtheit der Testelemente eines Testbands unter 5% liegt.

Um Streuverluste hinreichend zu reduzieren, ist es vorteilhaft, wenn die optische Trübung des Trägerbands und des Klebebandstücks im sichtbaren Wellenlängenbereich weniger als 10%, insbesondere etwa 8% beträgt. Günstig ist es auch, wenn die optische Trübung des Transportbands im sichtbaren Wellenlängenbereich weniger als 3%, insbesondere etwa 2,5% beträgt, und wenn die gesamte optische Trübung des Mehrschichtsystems im sichtbaren Wellenlängenbereich weniger als 20%, vorzugsweise etwa 15% beträgt.

Um herstellungsbedingte Störeinflüsse berücksichtigen zu können, sollte eine Trübungsabnahme des Mehrschichtsystems in einem gegebenen Zeitintervall insbesondere von etwa 1 bis 2 Wochen nach dessen Herstellung erfolgen, wobei anschließend die Trübung vergleichsweise konstant bleiben sollte.

Um Varianzen innerhalb einer gegebenen Bandeinheit hinreichend zu begrenzen, sollte die Trübungstoleranz für die Gesamtheit der Mehrschichtsysteme eines Testbands weniger als 5% betragen.

Vorteilhafterweise wird ein mehrschichtiges Klebebandstück eingesetzt, das aus einem beidseitig mit einer Klebemittelschicht versehenen transparenten Foliensubstrat besteht.

Für den vorgesehenen Einsatzzweck ist es auch günstig, wenn das Transportband und die Trägerfolie aus einem PET-Film bestehen. Allgemein sind Polymerfolien vorteilhaft einsetzbar. Beispielhaft sind als vorteilhafte Materialien neben Polyethylenterephtalat (PET) auch Polyvinylfluorid (PVF), Polyethylen (PE), Polypropylen (PP), Polyvinylidendifluorid (PVDF), Polyvinylchlorid (PVC), Polystyrol (PS), Ethen/Tetrafluorethylen-Copolymere (ETFE), Polycarbonat und Propylencarbonat zu nennen.

Für eine praxisgerechte Realisierung ist es auch von Vorteil, wenn die Dicke der Trägerfolie zwischen 20 und 25µm, die Dicke des Transportbands zwischen 10 und 15µm und die Dicke des Klebebandstücks zwischen 30 und 50µm beträgt.

Zur Berücksichtigung von Störeinflüssen, die sich durch einen spezifischen Aufbau ergeben, ist es vorteilhaft, wenn ein in der vorzugsweise durch eine Körperflüssigkeit, insbesondere Blut gebildeten Probensubstanz enthaltener Analyt durch eine relative Remissionsmessung bestimmbar ist, wobei den Testelementen Kalibrierdaten zugeordnet sind, welche die Konzentration des Analyten in Abhängigkeit von der gemessenen Remission definieren.

Ein weiterer Aspekt der Erfindung betrifft ein diagnostisches Messsystem, insbesondere für Blutzuckertests, mit einer erfindungsgemäßen diagnostischen Bandeinheit und einer auf die Rückseite der Reagenzschicht des in einer Messposition befindlichen Testelements ausgerichteten reflexionsphotometrischen Anordnung, die eine Lichtquelle und einen Photodetektor umfasst, wobei der Detektor außerhalb des direkten Reflexionspfades von der Lichtquelle durch das Mehrschichtsystem hindurch auf die Reagenzschicht eingestrahlten Messlichts angeordnet ist.

In diesem Zusammenhang ist es für eine Beleuchtungsoptimierung von Vorteil, wenn die Lichtquelle einen Leuchtfleck von weniger als 1 mm2 auf der Rückseite der Reagenzschicht erzeugt, wobei eine Körnung der Reagenzschicht als Streukörper dient.

Im Folgenden wird die Erfindung anhand des in der Zeichnung schematisch dargestellten Ausführungsbeispiels näher erläutert. Es zeigen:
- Fig. 1: eine diagnostische Bandeinheit in Form einer Bandkassette in geschnittener perspektivischer Darstellung;
- Fig. 2: das Testband der Bandkassette in einem ausschnittsweisen Längsschnitt im Bereich eines Testelements; und
- Fig. 3: eine auf das Testband ausgerichtete reflektometrische Messanordnung in schaubildlicher Darstellung.

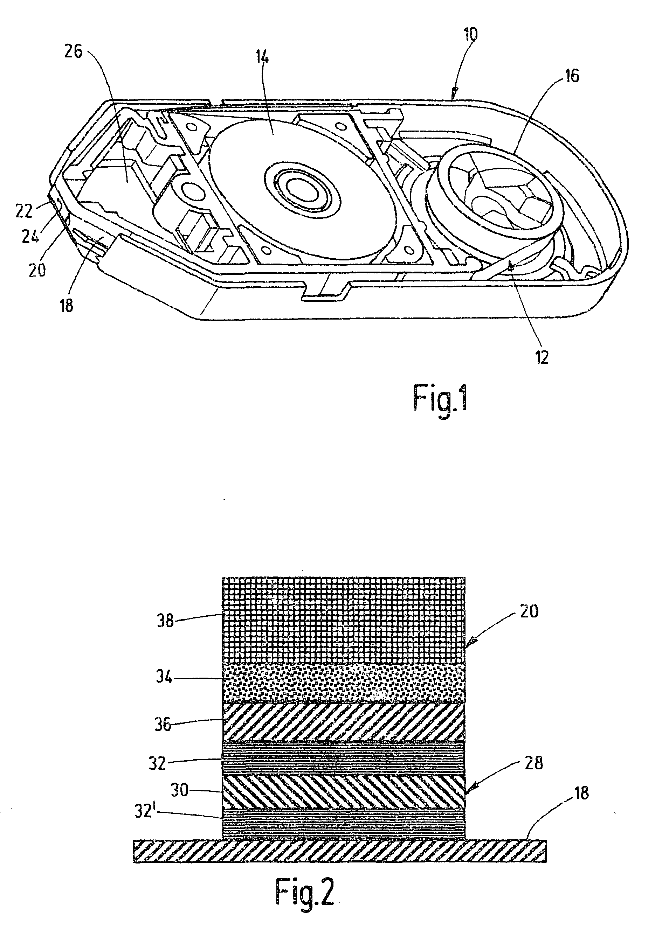

Die in Fig. 1 dargestellte Bandkassette 10 lässt sich als Verbrauchseinheit in ein nicht gezeigtes Handgerät zur Durchführung einer Vielzahl von Blutzucker-Selbsttests einsetzen. Die Kassette umfasst ein Testband 12, eine Abwickelspule 14 zum Abwickeln von ungebrauchtem Testband und eine Aufwickelspule 16 zum Aufwickeln von gebrauchtem Testband, wobei das Testband 12 ein aufrollbares Transportband 18 und eine Vielzahl von darauf im Abstand voneinander aufgebrachten Testelementen 20 aufweist.

Das unbenutzte Testband wird in Form eines Bandwickels auf der Abwickelspule 14 vor Umwelteinflüssen geschützt aufbewahrt. Das Testband 12 lässt sich über einen an der Aufwickelspule 16 angreifenden Drehantrieb vorspulen, so dass die Testelemente 20 an einer Applikationsstelle 22 für den Benutzer sukzessive bereitstellbar sind. Dort lässt sich an der freiliegenden Vorderseite 24 des jeweils aktiven Testelements 20 auf einfache Weise ein Blutstropfen auftragen, während durch eine in die Messkammer 26 der Kassette 10 eingreifende geräteseitige Messanordnung eine rückseitige reflexionsphotometrische Vermessung erfolgt. Der verbrauchte Testbandabschnitt wird auf der Aufwickelspule 16 entsorgt. Auf diese Weise können ca. 50 Tests verarbeitet werden, ohne dass ein Geräteeingriff oder umständliche Bedienschritte durch den Benutzer erforderlich wären.

Wie in Fig. 2 veranschaulicht, bildet das Transportband 18 mit den aufgebrachten Testelementen 20 einen mehrlagigen Verbundaufbau bzw. ein Mehrschichtsystem, dem ein vereinfachtes Herstellungsverfahren zugrunde liegt, wie es in der EP-A 1 593 434 beschrieben ist, auf die in diesem Zusammenhang Bezug genommen wird. Die Testelemente 20 werden dabei gleichsam als Testetiketten über ein doppelseitiges Klebebandstück 28 auf das Transportband 18 aufgeklebt. Das Klebebandstück 28 umfasst zu diesem Zweck eine Liner-Folie 30, die beidseitig mit einer Klebeschicht 32, 32' versehen ist. Die Nachweisreaktion erfolgt in einer dünnen Reagenzschicht 34, die auf eine Trägerfolie 36 als Trockensubstanz aufgebracht ist und darüber auf dem Klebebandstück 28 gehalten wird. Die Stärke einer Farbänderung des Reagenz steht dabei in einem funktionellen Zusammenhang mit der Konzentration des zu messenden Analyten (hier Blutglukose). An der Applikationsseite der Reagenzschicht 34 kann durch eine netzförmige Spreitschicht 38 eine flächige Verteilung der aufgetragenen Körperflüssigkeit erreicht werden.

Die Bestimmung der Glucosekonzentration erfolgt durch Remissionsphotometrie, wobei zur Berücksichtigung von konstanten Störgrößen ein relativer Remissionswert als Quotient aus dem Endwert und dem Startwert des Testelements 20 ermittelt wird. Dazu wird vor dem Probenauftrag der anfängliche Remissionswert des Testelementes gemessen und nach Aufgabe der Probe auf die Reagenzschicht nach einem Zeitintervall erneut die Remission gemessen. Dabei beinhalten sowohl die erste Messung als auch die nachfolgenden Messungen Störgrößen, die unabhängig von dem Nutzsignal Beiträge zum Messsignal liefern. Die Berechnung der Probenkonzentration erfolgt dann über eine im Gerät gespeicherte Rechenvorschrift (Funktionskurve). Diese Funktionskurve kann durch Kalibration des Messsystems festgelegt werden. Somit lassen sich die konstanten Störgrößen bei der Systemkalibration berücksichtigen.

Für eine zuverlässige Remissionsmessung müssen allerdings die optischen Störeinflüsse klein gegenüber dem Messsignal sein. Während bei herkömmlichen Teststreifen nur durch einen einlagigen Reagenzträger hindurch gemessen wird, sind in dem mehrschichtigen Verbundaufbau gemäß Fig. 1 verschiedene Folien und Klebeschichten zu berücksichtigen. In diesem Zusammenhang ist zu bemerken, dass alle auf dem Markt erhältlichen Folien Toleranzen bezüglich Ihrer optischen Spezifikationen aufweisen, die in den Herstellprozessen begründet sind. So wird der optische Strahlengang durch Transparenz, Absorption, Brechungsindex und Streuparameter jeder einzelnen Komponente und im Zusammenspiel der zur Verwendung kommenden Komponenten beeinflusst.

Um eine Remissionsmessung mit der bei Blutzuckermessungen erforderlichen Genauigkeit zu ermöglichen, lassen sich also nicht beliebige Folien einsetzen, sondern es sind in dem vorstehend beschriebenen mehrschichtigen Bandaufbau besondere Vorkehrungen und Abstimmungen hinsichtlich der optischen Eigenschaften zu treffen, wie sie nachstehend in Tabelle I zusammengefasst sind.

Mit abnehmender Transparenz bzw. Transmission der Folien verringert sich die Nutzsignalgröße. Daneben beeinflussen auch die Oberflächen- und Volumenstreuung die Signalqualität. Beides zusammen kann durch eine Trübungsmessung erfasst werden. Die genannten Parameter lassen sich mit einem standardisierten Messverfahren gemäß der Norm ASTM-D 1003-61 Methode A bestimmen (Standard Test Method for Haze and Luminous Transmittance of Transparent Plastics). Dabei wird die Streuung in Vorwärtsrichtung des durchgehenden Lichts mittels eines so genannten Hazemeters erfasst, wie es beispielsweise unter dem Handelsnamen "BYK Gardner Haze-Gard Plus" vertrieben wird. Die Funktionsweise dieses Geräts beruht darauf, dass ein kollimiertes Lichtbündel durch eine Folienprobe hindurch zentral in den Kugeleingang einer Ulbrichtkugel eingestrahlt wird, wobei an einem diametral zu dem Kugeleingang gegenüberliegenden Kugelausgang eine Lichtfalle angeordnet ist und die Detektion des Streulichtes unter einem Winkel von 90° mittig zu der Durchstrahlachse erfolgt. Somit kann zwischen ungestreutem Licht und an der Folientrübung in Vorwärtsrichtung gestreutem Licht unterschieden werden. Für die Transmissionsmessung wird dabei ein Kugelbereich mit einem Streustandard überdeckt.

Überraschend hat sich gezeigt, dass durch solche Haze-Messungen in Vorwärtsrichtung eine mit standardisierten Geräten erfassbare Einflussgröße auch für Remissionsmessungen in Rückwärtsrichtung gewonnen werden kann. Dabei wurde für einen Mehrschichtaufbau ein linearer Zusammenhang zwischen dem im diagnostischen Messsystem erfassten Remissions-Leerwert und dem im Hazemeter erfassten Materialparameter (Haze H) in Abhängigkeit von der Schichtdicke gefunden. Auf diese Weise ist es möglich, eine für das eigentliche Messsignal noch akzeptable Schwankung in der Streuung in eine definierte Toleranzbreite des Haze-Signals zu übertragen und dementsprechend eine Qualitätssicherung für die verwendeten Folien zu gewährleisten. Hierbei ist zu berücksichtigen, dass mit zunehmender Trübung auch die Schwankungs- bzw. Toleranzbreiten einzuschränken sind.

Das lange flexible Transportband 18 besteht vorteilhafterweise aus PET und besitzt eine Dicke d von etwa 12µm. Der Brechungsindex n beträgt 1,6. Es sollte weitgehend lichtdurchlässig bzw. transparent sein, wobei die spektrale Transmission T im Wellenlängenbereich zwischen 400 und 900nm mehr als 85% betragen sollte. Zweckmäßig sollte die Trübung (Haze H) bei etwa 2,5% liegen. Wie oben ausgeführt, ist die Einhaltung von Toleranzbereichen (Toleranzbreiten) von besonderer Wichtigkeit, um in einer Massenproduktion die Messpräzision innerhalb der zugelassenen Grenzen zu gewährleisten. Für das in der benötigten Länge abgeschnittene Transportband 18 sollte die Transmissionstoleranz ΔT weniger als 2% betragen, während die Trübung weniger als 0,5% variieren sollte.

Auch die übrigen Schichten in dem Mehrschichtsystem sind gemäß der Tabelle I speziell aufeinander abgestimmt.

**Tabelle I:**

| | d | n | T | ΔT | H | ΔH |
|---|---|---|---|---|---|---|
| Transportband 18 | 12 µm | 1,6 | >88% | <2% | 2,5% | <0,5% |
| Klebebandstück 28 | 42 µm | 1,5 | >85% | <3% | 8,0% | <3% |
| PET Trägerfolie 36 | 23 µm | 1,6 | >88% | <2% | 8,0% | <1% |
| Gesamtsystem 18,28,36 | 77 µm | 1,5 | >80% | <5% | 15% | <5% |

In der letzten Zeile der Tabelle sind die einheitlich ermittelten Messwerte für den gesamten Aufbau aus Transportband 18, Klebebandstück 28 und Trägerfolie 36 angegeben. Für das Gesamtsystem wurde eine besondere Problematik hinsichtlich der zeitlichen Konstanz der Messwerte dahingehend beobachtet, dass innerhalb der ersten 1 bis 2 Wochen nach der Herstellung eine Abnahme der Trübungswerte stattfand. Danach bleiben die Messwerte im Wesentlichen zeitlich stabil. Die Einzelkomponenten zeigen vor ihrem Zusammenfügen ein solches Verhalten nicht. Dies lässt sich dadurch erklären, dass die anfangs erhöhte Trübung auf den Einschluss von Luftbläschen beim Zusammenfügen der Einzelkomponenten zu dem Gesamtsystem und daraus resultierender erhöhter Streuung verursacht wird. Der zeitliche Abfall der gemessenen Trübungswerte wird dann durch das Entweichen der eingeschlossenen Luft durch Diffusion verursacht.

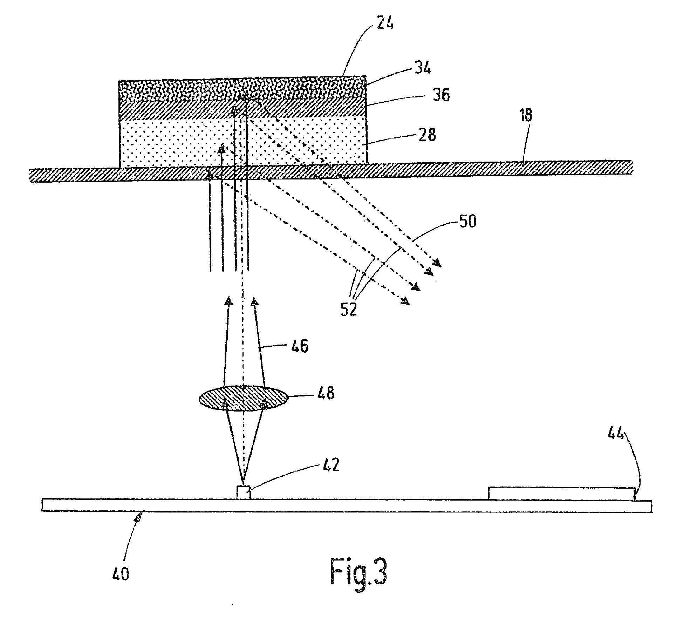

Fig. 3 veranschaulicht ein diagnostisches Messsystem, wie es in einem tragbaren Blutzuckermessgerät bei eingelegter Bandkassette 10 realisiert ist. Die rückseitige Vermessung der Reagenzschicht 34 erfolgt durch eine geräteseitige reflexionsphotometrische Anordnung 40, die eine Lichtquelle (LED 42) und einen optischen Detektor (Photodiode 44) umfasst. Das eingestrahlte Lichtbündel 46 wird über eine Optik (Sammellinse 48) auf einen kleinen Leuchtfleck auf der Rückseite der Reagenzschicht 44 fokussiert, wobei deren Körnung als Streukörper dient. Der Detektor 44 liegt dabei außerhalb des Ausfallwinkelbereichs des direkt reflektierten Lichtanteils, so dass im Wesentlichen nur das Streulicht erfasst wird.

Das System ist somit derart ausgelegt, dass das durch die Reagenzschicht 34 gebildete Testfeld mit hoher Intensität beleuchtet wird. Dabei tritt das Messlicht mit dem Reagenz in Wechselwirkung und wird in Abhängigkeit von Absorption und Transmission gestreut. Das gestreute Nutzlicht 50 fällt entsprechend dem Nachweisraumwinkel auf den Detektor 44. Dort wird allerdings auch das an den übrigen Komponenten 18, 28, 36 gestreute bzw. reflektierte Störlicht 52 nachgewiesen (der Strahlengang ist in Fig. 3 symbolisch vereinfacht). Die Güte des Messsignals ergibt sich daher aus dem Verhältnis von Nutz- zu Störlicht. Durch die beschriebene Anpassung der optischen Eigenschaften der im Strahlengang befindlichen Elemente bezüglich Brechungsindex, Transmission, Absorption und Streuvermögen im Bereich der Beleuchtungswellenlänge wird erreicht, dass das Messsignal der geforderten Güte entspricht.

Der funktionelle Zusammenhang zwischen der gemessenen Remission und der Konzentration des Analyten lässt sich durch eine Kalibrierkurve beschreiben. Das über eine Mikroelektronik gesteuerte Messgerät kann so zu jeder ermittelten Remission den korrekten Konzentrationswert zuordnen und über ein Display anzeigen. Die Bestimmung der Kalibrierkurve erfolgt an den gleichen Reagenzien in der beschriebenen Testfeldanordnung. Die sich durch den spezifischen Aufbau ergebenden Störlichtanteile können daher bei der Kalibrierung berücksichtigt werden. Die prozessbedingten Schwankungen der unterschiedlichen Störeinflüsse treten innerhalb einer gewissen Toleranz auf. Die zulässige Toleranz für diese Störeinflüsse ergibt sich aus den vorgegebenen bzw. erlaubten Grenzen auf Konzentrationsebene des betreffenden Analyten.

## Patentansprüche

1. Diagnostische Bandeinheit, insbesondere Bandkassette (10) für Blutzuckertests, mit einem als Bandwickel auf einer Spule aufgewickelten oder aufwickelbaren Testband (12), das aus einem Transportband (18) und einer Vielzahl von darauf aufgebrachten Testelementen (20) besteht, wobei die Testelemente (20) eine analytische Reagenzschicht (34), eine die Reagenzschicht (34) tragende Trägerfolie (36) und ein die Trägerfolie (36) mit dem Transportband (18) verbindendes Klebebandstück (28) aufweisen, wobei die Reagenzschicht (34) an ihrer von der Trägerfolie (36) abgewandten Vorderseite (24) zum Aufbringen einer Probensubstanz ausgebildet ist, und wobei die Testelemente (20) im Verbund mit dem lichtdurchlässigen Transportband (18) jeweils ein optisches Mehrschichtsystem für eine rückseitige reflexionsphotometrische Vermessung der Reagenzschicht (34) bilden, **dadurch gekennzeichnet, dass** der Brechungsindex und/oder die Transmission und/oder die Trübung der durch die Trägerfolie (36), das Klebebandstück (28) und das Transportband (18) gebildeten Schichten des Mehrschichtsystems innerhalb vorgegebener Toleranzen aufeinander abgestimmt sind.

2. Diagnostische Bandeinheit nach Anspruch 1, **dadurch gekennzeichnet, dass** der Brechungsindex des Transportbands (18), der Trägerfolie (36) und des Klebebandstücks (28) jeweils zwischen 1,4 und 1,7, vorzugsweise 1,5 bis 1,6 beträgt.

3. Diagnostische Bandeinheit nach Anspruch 1 oder 2, **dadurch gekennzeichnet, dass** die einzelnen Schichten des Mehrschichtsystems eine Brechungsindexdifferenz von maximal 0,2, vorzugsweise weniger als 0,1 aufweisen.

4. Diagnostische Bandeinheit nach einem der Ansprüche 1 bis 3, **dadurch gekennzeichnet, dass** der Gesamtbrechungsindex des Mehrschichtsystems 1,5 beträgt.

5. Diagnostische Bandeinheit nach einem der Ansprüche 1 bis 4, **dadurch gekennzeichnet, dass** das Transportband (18), das Klebebandstück (28) und die Trägerfolie (36) im sichtbaren Wellenlängenbereich eine Transmission von jeweils mehr als 80%, vorzugsweise 85% bis 92% besitzen.

6. Diagnostische Bandeinheit nach einem der Ansprüche 1 bis 5, **dadurch gekennzeichnet, dass** die Gesamttransmission des Mehrschichtsystems im sichtbaren Wellenlängenbereich mindestens 80% beträgt.

7. Diagnostische Bandeinheit nach einem der Ansprüche 1 bis 6, **dadurch gekennzeichnet, dass** die Transmissionstoleranz für die Gesamtheit der Testelemente (20) eines Testbands (12) unter 5% liegt.

8. Diagnostische Bandeinheit nach einem der Ansprüche 1 bis 7, **dadurch gekennzeichnet, dass** die optische Trübung der Trägerfolie (36) und des Klebebandstücks (28) im sichtbaren Wellenlängenbereich weniger als 10%, insbesondere etwa 8% beträgt.

9. Diagnostische Bandeinheit nach einem der Ansprüche 1 bis 8, **dadurch gekennzeichnet, dass** die optische Trübung des Transportbands (18) im sichtbaren Wellenlängenbereich weniger als 3%, insbesondere etwa 2,5% beträgt.

10. Diagnostische Bandeinheit nach einem der Ansprüche 1 bis 9, **dadurch gekennzeichnet, dass** die gesamte optische Trübung des Mehrschichtsystems im sichtbaren Wellenlängenbereich weniger als 20%, vorzugsweise etwa 15% beträgt.

11. Diagnostische Bandeinheit nach einem der Ansprüche 1 bis 10, **dadurch gekennzeichnet, dass** die Trübungstoleranz für die Gesamtheit der Mehrschichtsysteme eines Testbands (12) weniger als 5% beträgt.

12. Diagnostische Bandeinheit nach einem der Ansprüche 1 bis 11, **dadurch gekennzeichnet, dass** das mehrschichtige Klebebandstück (28) aus einem beidseitig mit einer Klebemittelschicht (32,32') versehenen transparenten Foliensubstrat (30) besteht.

13. Diagnostische Bandeinheit nach einem der Ansprüche 1 bis 12, **dadurch gekennzeichnet, dass** das Transportband (18) und die Trägerfolie (36) aus einem PET-Film bestehen.

14. Diagnostisches Messsystem, insbesondere für Blutzuckertests mit einer diagnostischen Bandeinheit (10) nach einem der vorhergehenden Ansprüche und einer auf die Rückseite der Reagenzschicht (34) des in einer Messposition befindlichen Testelements (20) ausgerichteten reflexionsphotometrischen Anordnung (40), die eine Lichtquelle (42) und einen Photodetektor (44) umfasst, wobei der Photodetektor (44) außerhalb des direkten Reflexionspfades von der Lichtquelle (42) durch das Mehrschichtsystem hindurch auf die Reagenzschicht (34) eingestrahlten Messlichts angeordnet ist.

15. Diagnostisches Messsystem nach Anspruch 14, **dadurch gekennzeichnet, dass** die Lichtquelle (42) einen Leuchtfleck von weniger als 1 mm2 auf der Rückseite der Reagenzschicht (34) erzeugt, wobei eine Körnung der Reagenzschicht (34) als Streukörper dient.

## Claims

1. Diagnostic tape unit, in particular a tape cassette (10) for blood sugar tests with a test tape (12) that is wound onto or can be wound onto a spool as a tape reel, the test tape consisting of a transport tape (18) and a plurality of test elements (20) mounted thereon, where the test elements (20) have an analytical reagent layer (34), a carrier foil (36) supporting the reagent layer (34) and a piece of adhesive tape (28) connecting the carrier foil (36) with the transport tape (18), where the front side (24) of the reagent layer (34) facing away from the carrier foil (36) is designed for the application of a sample substance, and where the test elements (20) in combination with the light transmitting transport tape (18) in each case form an optical multi-layer system for a rear-side reflection-photometric measurement of the reagent layer (34), **characterized in that** the refractive index and/or the transmission and/or the haze of the layers of the multi-layer system formed by the carrier foil (36), the piece of adhesive tape (28) and the transport tape (18) are matched within predetermined tolerances.

2. Diagnostic tape unit according to claim 1, **characterized in that** the refractive index of the transport tape (18), the carrier tape (36) and the piece of adhesive tape (28) is in each case between 1.4 and 1.7, preferably 1.5 to 1.6.

3. Diagnostic tape unit according to claim 1 to 2, **characterized in that** the individual layers of the multi-layer system have a maximum refractive index difference of 0.2 and preferably of less than 0.1.

4. Diagnostic tape unit according to one of the claims 1 to 3, **characterized in that** the total refractive index of the multi-layer system is 1.5.

5. Diagnostic tape unit according to one of the claims 1 to 4, **characterized in that** the transport tape (18), the piece of adhesive tape (28) and the carrier foil (36) each have a transmission in the visible wavelength range of more than 80 %, preferably 85 % to 92%.

6. Diagnostic tape unit according to one of the claims 1 to 5, **characterized in that** the total transmission of the multi-layer system in the visible wavelength range is at least 80%.

7. Diagnostic tape unit according to one of the claims 1 to 6, **characterized in that** the transmission tolerance is less than 5 % for the entirety of the test elements (20) of a test tape (12).

8. Diagnostic tape unit according to one of the claims 1 to 7, **characterized in that** the optical haze of the carrier foil (36) and of the piece of adhesive tape (28) in the visible wavelength range is less than 10 %, in particular about 8 %.

9. Diagnostic tape unit according to one of the claims 1 to 8, **characterized in that** the optical haze of the transport tape (18) in the visible wavelength range is less than 3 %, in particular about 2.5 %.

10. Diagnostic tape unit according to one of the claims 1 to 9, **characterized in that** the total optical haze of the multi-layer system in the visible wavelength range is less than 20 %, preferably about 15 %.

11. Diagnostic tape unit according to one of the claims 1 to 10, **characterized in that** the haze tolerance for the entirety of the multi-layer systems of a test tape (12) is less than 5 %.

12. Diagnostic tape unit according to one of the claims 1 to 11, **characterized in that** the multi-layer piece of adhesive tape (28) consists of a transparent foil substrate (30) furnished on both sides with an adhesive layer (32, 32').

13. Diagnostic tape unit according to one of the claims 1 to 12, **characterized in that** the transport tape (18) and the carrier foil (36) consist of a PET film.

14. Diagnostic measuring system, in particular for blood sugar tests with a diagnostic tape unit (10) according to one of the previous claims and a reflection photometric arrangement (40) oriented towards the rear side of the reagent layer (34) of the test element (20) located in a measuring position which comprises a light source (42) and a photodetector (44) where the photodetector (44) is arranged outside the direct reflection path of the measuring light radiated by the light source (42) through the multi-layer system onto the reagent layer (34).

15. Diagnostic measuring system according to claim 14, **characterized in that** the light source (42) generates a light spot of less than 1 mm² on the rear side of the reagent layer (34) where a granularity of the reagent layer (34) serves as a scattering body.

## Revendications

1. Ensemble bande diagnostique, notamment cassette à bande (10) pour tests de glycémie, comprenant une bande de test (12) enroulée ou pouvant être enroulée en tant qu'enroulement de bande sur une bobine, laquelle bande de test est formée d'une bande de transport (18) et d'une pluralité d'éléments de test (20) disposés sur celle-ci, les éléments de test (20) comprenant une couche réactive analytique (34), un film de support (36) portant la couche réactive (34) et une pièce adhésive (28) reliant le film de support (36) à la bande de transport (18), la couche réactive (34) étant réalisée pour déposer un échantillon de substance sur sa face avant (24) opposée au film de support (36), et les éléments de test (20) formant à chaque fois, en association avec la bande de transport (18) translucide, un système optique multicouche pour effectuer une mesure photométrique par réflexion sur la face arrière de la couche réactive (34), **caractérisé en ce que** l'indice de réfraction et/ou la transmission et/ou la turbidité des couches du système multicouche formées par le film de support (36), la pièce adhésive (28) et la bande de transport (18) sont modulés les uns par rapport aux autres dans les limites de tolérances prédéfinies.

2. Ensemble bande diagnostique selon la revendication 1, **caractérisé en ce que** l'indice de réfraction de la bande de transport (18), du film de support (36) et de la pièce adhésive (28) est compris à chaque fois entre 1,4 et 1,7, de préférence entre 1,5 et 1,6.

3. Ensemble bande diagnostique selon la revendication 1 ou 2, **caractérisé en ce que** les couches individuelles du système multicouche présentent une différence d'indice de réfraction de 0,2 au maximum, de préférence de moins de 0,1.

4. Ensemble bande diagnostique selon l'une des revendications 1 à 3, **caractérisé en ce que** l'indice de réfraction total du système multicouche est égal à 1,5.

5. Ensemble bande diagnostique selon l'une des revendications 1 à 4, **caractérisé en ce que** la bande de transport (18), la pièce adhésive (28) et le film de support (36) présentent une transmission à chaque fois supérieure à 80%, de préférence entre 85% et 92%, dans le domaine de longueurs d'ondes visibles.

6. Ensemble bande diagnostique selon l'une des revendications 1 à 5, **caractérisé en ce que** la transmission totale du système multicouche dans le domaine de longueurs d'ondes visibles est d'au moins 80%.

7. Ensemble bande diagnostique selon l'une des revendications 1 à 6, **caractérisé en ce que** la tolérance de transmission pour l'ensemble des éléments de test (20) d'une bande de test (12) est inférieure à 5%.

8. Ensemble bande diagnostique selon l'une des revendications 1 à 7, **caractérisé en ce que** la turbidité optique du film de support (36) et de la pièce adhésive (28) dans le domaine de longueurs d'ondes visibles est inférieure à 10%, notamment égale à environ 8%.

9. Ensemble bande diagnostique selon l'une des revendications 1 à 8, **caractérisé en ce que** la turbidité optique de la bande de transport (18) dans le domaine de longueurs d'ondes visibles est inférieure à 3%, notamment égale à environ 2,5%.

10. Ensemble bande diagnostique selon l'une des revendications 1 à 9, **caractérisé en ce que** la turbidité optique totale du système multicouche dans le domaine de longueurs d'ondes visibles est inférieure à 20%, notamment égale à environ 15%.

11. Ensemble bande diagnostique selon l'une des revendications 1 à 10, **caractérisé en ce que** la tolérance de turbidité pour l'ensemble des systèmes multicouches d'une bande de test (12) est inférieure à 5%.

12. Ensemble bande diagnostique selon l'une des revendications 1 à 11, **caractérisé en ce que** la pièce adhésive multicouche (28) est formée d'un substrat en forme de feuille (30) translucide pourvu d'une couche d'adhésif (32, 32') sur les deux côtés.

13. Ensemble bande diagnostique selon l'une des revendications 1 à 12, **caractérisé en ce que** la bande de transport (18) et le film de support (36) sont formés d'un film en PET.

14. Système de mesure diagnostique, notamment pour des tests de glycémie, comprenant un ensemble bande diagnostique (10) selon l'une des revendications précédentes et un dispositif de photométrie par réflexion (40) orienté vers la face arrière de la couche réactive (34) de l'élément de test (20) se trouvant dans une position de mesure, dispositif qui comprend une source lumineuse (42) et un photodétecteur (44), le photodétecteur (44) étant situé hors du trajet de réflexion direct du faisceau de lumière de mesure émis de la source lumineuse sur la couche réactive (34) à travers le système multicouche.

15. Système de mesure diagnostique selon la revendication 14, **caractérisé en ce que** la source lumineuse (42) produit un spot lumineux de moins de 1 mm² sur la face arrière de la couche réactive (34), une granularité de la couche réactive (34) servant de corps de dispersion.
